(19) Europäisches Patentamt
European Patent Office
Office européen des brevets

(11) **EP 3 766 502 B1**

(12) **EUROPEAN PATENT SPECIFICATION**

(45) Date of publication and mention of the grant of the patent:
**09.09.2026 Bulletin 2026/37**

(21) Application number: **19767269.4**

(22) Date of filing: **13.03.2019**

(51) International Patent Classification (IPC):
***A61K 31/7068*** *(2006.01)* ***A61K 31/555*** *(2006.01)*
***A61K 33/243*** *(2019.01)* ***A61K 31/282*** *(2006.01)*
***A61K 45/00*** *(2006.01)* ***A61P 35/00*** *(2006.01)*
***A61P 35/02*** *(2006.01)*

(52) Cooperative Patent Classification (CPC):
(C-Sets available)
**A61K 33/243; A61K 31/282; A61K 31/555; A61K 31/7068; A61K 45/00; A61P 35/00; A61P 35/02** (Cont.)

(86) International application number:
**PCT/JP2019/010169**

(87) International publication number:
**WO 2019/176984 (19.09.2019 Gazette 2019/38)**

(54) **ANTITUMOR AGENT, ANTITUMOR EFFECT POTENTIATOR AND ANTITUMOR KIT COMPRISING AN ANTITUMOR PLATINUM COMPLEX; AND1-(2-DEOXY-2-FLUORO-4-THIO-(3-D-ARABINOFURANOSYL)CYTOSINE**

ANTITUMORMITTEL, ANTITUMORWIRKUNGSVERSTÄRKER UND ANTITUMORKIT, UMFASSEND EINEN ANTITUMORPLATINKOMPLEX; UND 1-(2-DESOXY-2-FLUOR-4-THIO-(3-D-ARABINOFURANOSYL)CYTOSIN

AGENT ANTITUMORAL, POTENTIATEUR D'EFFET ANTITUMORAL ET KIT ANTITUMORAL COMPRENANT UN COMPLEXE DE PLATINE ANTITUMORAL ; ET DE LA 1-(2-DÉSOXY-2-FLUORO-4-THIO-(3-D-ARABINOFURANOSYL)CYTOSINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.03.2018 JP 2018045430**

(43) Date of publication of application:
**20.01.2021 Bulletin 2021/03**

(73) Proprietor: **FUJIFILM Corporation**
**Tokyo 106-8620 (JP)**

(72) Inventors:
- **YAMADA Takayuki**
**Ashigarakami-gun, Kanagawa 258-8577 (JP)**
- **SAEKI Kazunori**
**Ashigarakami-gun, Kanagawa 258-8577 (JP)**
- **UEMATSU Rena**
**Ashigarakami-gun, Kanagawa 258-8577 (JP)**
- **MORIMURA Kaoru**
**Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**EP-A1- 0 841 344**
**EP-A1- 2 832 740**
**EP-A1- 2 883 542**
**WO-A1-2018/043530**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **ZAJCHOWSKI ET AL.: "Anti-tumor efficacy of the nucleoside analog 1-(2-deoxy-2-fluoro-4-thio-[beta]-D-arabinofuranosyl) cytosine (4'-thio-FAC) in human pancreatic and ovarian xenograft models. | Cancer Research", S PROC AMER ASSOC CANCER RES, VOLUME 45, ABSTRACT 3087, 1 April 2004 (2004-04-01), XP055687952, Retrieved from the Internet <URL:https://cancerres.aacrjournals.org/content/64/7_Supplement/714.3> [retrieved on 20200421]**
- **FALCHOOK GERALD STEVEN ET AL: "Abstract CT100: First-in-human phase 1 trial of pyrimidine anti-metabolite FF-10502-01 in patients with advanced cancer LNKD - doi:10.1158/1538-7445.AM2017-CT100", PROCEEDINGS: AACR ANNUAL MEETING, 1 April 2017 (2017-04-01), pages 1 - 4, XP055787953, Retrieved from the Internet <URL:https://cancerres.aacrjournals.org/content/77/13_Supplement/CT100> [retrieved on 20210319], DOI: 10.1158/1538-7445.AM2017-CT100Published**
- **DILRUBA SHAHANA ET AL: "Platinum-based drugs: past, present and future", CANCER CHEMOTHERAPY AND PHARMACOLOGY, SPRINGER VERLAG , BERLIN, DE, vol. 77, no. 6, 17 February 2016 (2016-02-17), pages 1103 - 1124, XP035873013, ISSN: 0344-5704, [retrieved on 20160217], DOI: 10.1007/S00280-016-2976-Z**
- **ZAJCHOWSKI, D. ET AL.: "Anti-tumor efficacy of the nucleoside analog 1-(2-deoxy-2-fluoro-4-thio-p-D- arabinofuranosyl) cytosine (4'-thio-FAC) in human pancreatic and ovarian xenograft models", PROCEEDINGS OF THE AMERICAN ASSOCIATION FOR CANCER RESEARCH ANNUAL MEETING, vol. 114, no. 6, 2004, pages 1002 - 1009, XP055213460**
- **HIRORO AKAMATSU; NOBUYUKI YAMAMOTO: "Cell-killing antineoplastic drug: Platinum preparations, alkylating drugs, anticancer antibiotics", JAPANESE JOURNAL OF CLINICAL MEDICINE, vol. 72, no. 2, 30 November 2013 (2013-11-30), pages 124 - 126, XP009523356, ISSN: 0047-1852**
- **MIURA, S. ET AL.: "The antitumor mechanism of 1- (2-deoxy-2-fluoro-4-thio-/beta-D-arabinofuranosyl)- cytosine: effects of its triphosphate on mammalian DNA polymerases", JAPANESE JOURNAL OF CANCER RESEARCH, vol. 92, no. 5, 2001, pages 562 - 567, XP055213494, DOI: 10.1111/j.1349-7006.2001.tb01130.x**
- **MIMA, S. ET AL.: "FF-10502, an Antimetabolite with Novel Activity on Dormant Cells, Is Superior to Gemcitabine for Targeting Pancreatic Cancer Cells", THE JOURNAL OF PHARMACOLOGY AND EXPERIMENTAL THERAPEUTICS, vol. 366, no. 1, July 2018 (2018-07-01), pages 125 - 135, XP55636832**



(52) Cooperative Patent Classification (CPC): (Cont.)

C-Sets
**A61K 31/282, A61K 2300/00;**
**A61K 31/555, A61K 2300/00;**
**A61K 31/7068, A61K 2300/00;**
**A61K 33/243, A61K 2300/00**

## Description

Field of the Invention

The present invention relates to an antitumor agent, an antitumor effect enhancer, and an antitumor kit for use in the treatment of a tumor as defined in the claims.

Related Art

It is known that 1-(2-deoxy-2-fluoro-4-thio-β-D-arabinofuranosyl)cytosine (hereinafter, sometimes referred to as "Compound A") has an excellent antitumor activity and is therefore useful as an antitumor agent (Patent Document 1). It is also known that Compound A has a potent antitumor activity even in case of being orally administered to mice (Non-Patent Documents 1 and 2). In addition, a salt of Compound A and a method for producing the same are also known (Patent Documents 2 to 4). Furthermore, an antineoplastic drug having an enhanced antitumor effect by combining a specific acylthiourea compound with an antitumor agent (paclitaxel, gemcitabine, lapatinib, a tegafur/gimeracil/oteracil potassium combination drug, irinotecan, or the like) is known (Patent Document 5). Non-Patent Document 6 describes an anti-tumor effect of Compound A in combination with taxol or carboplatin in ES-2 and SK-OV-3 ovarian cancer cells or in combination with gemcitabine in pancreatic cancer cells.

In cancer chemotherapy, an antitumor platinum complex is known as a representative drug for antitumor agents. In addition, preparation including an antitumor platinum complex as an active ingredient is known as platinum preparation. Representative examples of the antitumor platinum complex include cisplatin, carboplatin, oxaliplatin, and nedaplatin, which are used for the treatment of various malignant tumors. However, these antitumor platinum complexes are likely to induce high renal toxicity and resistant cancer, which poses a problem in a clinical setting (Non- Patent Document 3).

In a clinical setting, a multidrug combination therapy is being carried out for the purpose of compensating for the difference in the sensitivity of each antitumor agent to the tumor and of enhancing the drug efficacy, and antitumor platinum complexes are also often used in combination therapy with other antitumor agents. In particular, there is known a combination therapy with taxanes such as paclitaxel and docetaxel; camptothecins such as irinotecan and nogitecan; nucleic acid antimetabolites such as gemcitabine, 5-fluorouracil, capecitabine, and a tegafur/gimeracil/oteracil calcium mixture (S-1); or topoisomerase II inhibitors such as etoposide and doxorubicin, which is used as a therapeutic regimen for a wide range of solid cancers.

For example, a combination of gemcitabine with cisplatin to a pancreatic cancer patient has a response rate of 11.5% and a median survival time of 7.5 months (Non- Patent Document 4) and a combination of gemcitabine with oxaliplatin to a pancreatic cancer patient has a median survival time of 5.7 months and a response rate of 9% (Non- Patent Document 5), and therefore it cannot be said that the therapeutic effect is sufficiently high in any combination therapy with an antitumor platinum complex.

Prior Art Documents

Patent Documents

Patent Document 1: WO1997/038001A
Patent Document 2: WO2013/146833A
Patent Document 3: WO2011/074484A
Patent Document 4: WO2014/027658A
Patent Document 5: WO2013/100014A


Non-Patent Documents

Non-Patent Document 1: Cancer Letters, 1999, Vol. 144, pp. 177 to 182
Non-Patent Document 2: Oncology Reports, 2002, Vol. 9, pp. 1319 to 1322
Non-Patent Document 3: YAKUGAKU ZASSHI, 2008, Vol. 128, pp. 307 to 316
Non-Patent Document 4: Journal of Clinical Oncology, 2006, Vol. 24, pp. 3946 to 3952
Non-Patent Document 5: Journal of Clinical Oncology, 2009, Vol. 23, pp. 3778 to 3785
Non-Patent Document 6: Cancer Research, Proc. Amer. Assoc. Cancer Res., 2004, Vol. 45, Abstract 3087

## SUMMARY OF THE INVENTION

In recent years, combination therapy has been widely carried out rather than administering an antitumor agent alone. However, it is not entirely clear whether any antitumor agent is used in combination to enhance or offset the antitumor effect thereof.

An object according to the invention is to provide an antitumor agent, an antitumor kit, and an antitumor effect enhancer for use in the treatment of a tumor as defined in the claims which have a superior antitumor effect as compared with gemcitabine, an antitumor platinum complex (platinum preparation) and a combination therapy thereof.

In view of the above, the present inventors have studied combination use of various drugs, and as a result, have found that combination use of an antitumor platinum complex and Compound A exhibits a significant antitumor effect. The invention has been completed based on these findings.

Compound A or a salt thereof exhibits a significant antitumor effect in case of being used in combination with an antitumor platinum complex. That is, the antitumor agent and antitumor kit according to the aspects of the invention as defined in the claims have a superior antitumor effect as compared with gemcitabine alone, an antitumor platinum complex alone, or a combination of gemcitabine and an antitumor platinum complex. The antitumor effect enhancer according to the aspect of the invention as defined in the claims can be administered in combination with an antitumor platinum complex to thereby enhance an antitumor effect.

## BRIEF DESCRIPTION OF THE DRAWINGS

Fig. 1 is a graph showing a combinational effect of Compound A and cisplatin on cell viability of a human pancreatic cancer-derived cell line SUIT-2.

Fig. 2 is a graph showing the combinational effect of Compound A and oxaliplatin on the cell viability of the human pancreatic cancer-derived cell line SUIT-2.

Fig. 3 is a graph showing the combinational effect of Compound A and cisplatin on the cell viability of a human ovarian cancer cell line ES-2.

Fig. 4 is a graph showing the combinational effect of Compound A and cisplatin on the cell viability of a human ovarian cancer cell line SK-OV-3.

Fig. 5 is a graph showing the combinational effect of Compound A and carboplatin on the cell viability of the human ovarian cancer cell line SK-OV-3.

Fig. 6 is a graph showing the combinational effect of Compound A and carboplatin on the cell viability of the human ovarian cancer cell line ES-2.

Fig. 7 is a graph showing the combinational effect of Compound A and cisplatin on the cell viability of a human cholangiocarcinoma-derived cell line HuCC-T1.

Fig. 8 is a graph showing the combinational effect of Compound A and cisplatin on the cell viability of a human cholangiocarcinoma-derived cell line TFK-1 in spheroid culture.

Fig. 9 is a graph showing the combinational effect of Compound A and cisplatin on the cell viability of the human cholangiocarcinoma-derived cell line HuCC-T1 in spheroid culture.

Fig. 10 is a graph showing the combinational effect of Compound A and cisplatin on the cell viability of a human breast cancer-derived cell line HCC1954 in spheroid culture.

## DESCRIPTION OF THE PREFERRED EMBODIMENTS

In addition, the range expressed by "to" includes the values at both ends of "to" unless otherwise specified.

The term "subject" is a mammal such as a human, a mouse, a monkey, or a livestock in need of prevention or treatment thereof, preferably a human in need of prevention or treatment thereof.

The term "preventing" refers to inhibition of disease onset, reduction of disease onset risk, delay of disease onset, or the like.

The term "treating" refers to improvement of, inhibition (maintenance or delay) of, or the like of progression of a target disease or condition.

The term "treatment" refers to preventing, treating, or the like of a variety of diseases. The term "tumor" refers to a benign tumor or a malignant tumor.

The term "benign tumor" refers to a tumor in which a tumor cell and a sequence thereof have a morphology close to that of a normal cell from which such a tumor cell is derived, and which is not invasive or metastatic.

The term "malignant tumor" refers to a tumor in which the morphology and sequence of a tumor cell are different from those of a normal cell from which such a tumor cell is derived, and which is invasive or metastatic.

Hereinafter, the invention will be described in detail.

The invention relates to an antitumor agent for use in the treatment of a tumor as defined in the claims including an antitumor platinum complex and 1-(2-deoxy-2-fluoro-4-thio-β-D-arabinofuranosyl)cytosine (Compound A) or a salt thereof. In addition, the invention relates to an antitumor agent for use in the treatment of a tumor as defined in the claims including an antitumor platinum complex and Compound A or a salt thereof in combination.

In the invention, the term "combination" refers to a combination for use of compounds in combination and includes both a form in which separate substances are used in combination at the time of administration and a form as a mixture thereof (combination drug). In the invention, the term "combination" does not mean that the compound of the invention and the antitumor platinum complex are administered at exactly the same administration time. As long as one administration schedule includes an aspect in which the compound of the invention and the antitumor platinum complex are administered, the form in which they are administered simultaneously or separately means "combination". In case of being administered separately, the compound of the invention may be administered first, followed by the antitumor platinum complex. Alternatively, the compound may be administered after the antitumor platinum complex has been administered first.

First, Compound A or a salt thereof will be described.

The salt may be, for example, a pharmaceutically acceptable salt and specific examples thereof include a mineral acid salt, an organic carboxylate, and a sulfonate. Preferred examples of the salt include a mineral acid salt and a sulfonate.

Examples of the mineral acid salt include hydrochloride, hydrobromide, hydroiodide, nitrate, phosphate, and sulfate, among which hydrochloride, hydroiodide, nitrate, or sulfate is preferable, and hydrochloride is more preferable. Examples of the organic carboxylate include formate, acetate, citrate, oxalate, fumarate, maleate, succinate, malate, tartrate, aspartate, trichloroacetate, and trifluoroacetate. Examples of the sulfonate include methanesulfonate, benzenesulfonate, p-toluenesulfonate, mesitylenesulfonate, and naphthalenesulfonate, among which methanesulfonate is preferable.

The salt of Compound A may be an anhydride, a hydrate, or a solvate. In case where the term "salt" is simply used in the present specification, the salt may be in the form of an anhydride, a hydrate, or a solvate. As for the term "anhydride" used in the present specification, it refers to the salt in a state where it is not a hydrate or a solvate, unless otherwise stated. Even though it is a substance which originally does not form a hydrate or a solvate, the salt of Compound A which does not have crystallization water, hydration water and an interacting solvent is also included in the "anhydride" referred to in the invention. The anhydride may also be referred to as "anhydrate". In case where the salt of Compound A is a hydrate, the number of hydration water molecules is not particularly limited, and the hydrate may be a monohydrate, a dihydrate, or the like. Examples of the solvate include methanol solvate, ethanol solvate, propanol solvate, and 2-propanol solvate.

Specific examples of a particularly preferred salt of Compound A are as follows:

methanesulfonate of 1-(2-deoxy-2-fluoro-4-thio-β-D-arabinofuranosyl)cytosine;
hydrochloride of 1-(2-deoxy-2-fluoro-4-thio-β-D-arabinofuranosyl)cytosine; and
1/2 sulfate of 1-(2-deoxy-2-fluoro-4-thio-β-D-arabinofuranosyl)cytosine;
nitrate of 1-(2-deoxy-2-fluoro-4-thio-β-D-arabinofuranosyl)cytosine; and
hydroiodide of 1-(2-deoxy-2-fluoro-4-thio-β-D-arabinofuranosyl)cytosine; as well as anhydrides of the foregoing salts.

In the invention, Compound A or a salt thereof may be used alone or in combination of two or more thereof.

Next, a method for producing Compound A or a salt thereof will be described. Compound A can be produced, for example, by the method described in WO1997/038001A or Journal of Organic Chemistry, 1999, Vol. 64, pp. 7912 to 7920. The salt of Compound A or a hydrate or solvate thereof can be produced, for example, by the method described in WO2014/027658A.

Compound A or a salt thereof according to the embodiment of the invention can be used as an antitumor agent or as an active ingredient of a pharmaceutical composition.

(Antitumor platinum complex)

In the invention, the antitumor platinum complex (platinum complex having an antitumor effect) may be, for example, cisplatin, carboplatin, oxaliplatin, nedaplatin, zeniplatin, enloplatin, ormaplatin, loboplatin, sebriplatin, lobaplatin, miboplatin, or spiroplatin.

The antitumor platinum complex of the embodiment of the invention is preferably cisplatin, carboplatin, oxaliplatin or nedaplatin, and more preferably cisplatin, carboplatin or oxaliplatin.

These antitumor platinum complexes can be produced by known methods.

In addition, these antitumor platinum complexes can also be available by purchasing a commercially available

product thereof. For example, cisplatin is commercially available as Randa (registered trademark) from Nippon Kayaku Co., Ltd., Platosin (registered trademark) from Pfizer Inc., Cisplamerck (registered trademark) from Merck & Co., Inc., and Briplatin (registered trademark) from Bristol-Myers Squibb Company. Carboplatin is commercially available as Paraplatin (registered trademark) from Bristol-Myers Squibb Company and Carbomerck (registered trademark) from Merck & Co., Inc. Oxaliplatin is commercially available as Elplat (registered trademark) or Eloxatin (registered trademark) from Yakult Honsha Co., Ltd. Nedaplatin is commercially available as Aqupla (registered trademark) from Nichi-Iko Pharmaceutical Co., Ltd. Miboplatin is commercially available as miboplatin hydrochloride or Lobaplatin (registered trademark) from Chugai Pharmaceutical Co., Ltd.

In the invention, the antitumor platinum complex may form a pharmaceutically acceptable salt (hereinafter, sometimes referred to as a "salt thereof") with an acid or a base. The antitumor platinum complex of the embodiment of the invention also includes pharmaceutically acceptable salts thereof. The antitumor platinum complex may be used alone or in combination of two or more thereof. In addition to the antitumor platinum complex or the salt thereof, the antitumor platinum complex may be a composition including the antitumor platinum complex or the salt thereof.

The salt may be, for example, a pharmaceutically acceptable salt and specific examples thereof include salts of commonly known basic groups such as amino group, and salts of commonly known acidic groups such as hydroxyl group and carboxyl group.

Examples of salts in basic groups include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylene sulfonic acid, and naphthalene sulfonic acid.

Examples of salts in acidic groups include salts with alkali metals such as sodium and potassium; salts with alkaline earth metals such as calcium and magnesium; ammonium salts; and salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine.

Compound A is an antitumor agent having an excellent DNA synthesis inhibitory effect. In case where Compound A is used in combination with an antitumor platinum complex, it is expected that such a combination will have an effect of enhancing the antitumor effect of the antitumor platinum complex without showing a significant increase in toxicity.

(Antitumor agent)

According to the invention, there are provided an antitumor agent for use in the treatment of a tumor as defined in the claims including an antitumor platinum complex and 1-(2-deoxy-2-fluoro-4-thio-β-D-arabinofuranosyl)cytosine or a salt thereof; and an antitumor agent including 1-(2-deoxy-2-fluoro-4-thio-β-D-arabinofuranosyl)cytosine or a salt thereof, each of which is used in combination with an antitumor platinum complex.

Typically, the antitumor agent for use in the treatment of a tumor as defined in the claims according to the embodiment of the invention may contain additives such as an excipient, a binder, a lubricant, a disintegrant, a coloring agent, a flavoring agent, an emulsifier, a surfactant, a solubilizing agent, a suspending agent, an isotonic agent, a buffering agent, a preservative, an antioxidant, a stabilizer, and an absorption promoter, which have been commonly used in the formulation.

The antitumor agent for use in the treatment of a tumor as defined in the claims according to the embodiment of the invention including an antitumor platinum complex and Compound A or a salt thereof may be a one-part preparation including an antitumor platinum complex and Compound A or a salt thereof or may be a two-part preparation including an antitumor platinum complex and Compound A or a salt thereof. Preferably, the antitumor agent according to the embodiment of the invention is a two-part preparation in which an antitumor platinum complex and Compound A or a salt thereof are formulated into separate preparations.

In case where an antitumor platinum complex and Compound A or a salt thereof are used as separate preparations, individual preparations can be administered to a subject simultaneously, separately, sequentially, or at intervals. In addition, the route for administering a composition including an antitumor platinum complex and the route for administering a composition including Compound A may be the same as or different from each other (for example, oral administration and injection).

The route of administration of the antitumor agent according to the embodiment of the invention includes intravenous, intraarterial, intrarectal, intraperitoneal, intramuscular, intratumoral or intravesical injection, oral administration, transdermal administration and/or suppository.

Parenteral administration is preferable as the route of administration. For example, intravenous injections (intravenous infusions) such as drip infusion, intramuscular injection, intraperitoneal injection, subcutaneous injection, intraocular injection and/or intrathecal injection can be mentioned as parenteral administration. The method of admin-

istration includes administration by syringe or drip infusion.

With respect to the dose and administration method of Compound A or a salt thereof, a dose of 1 to 2000 $mg/m^2$/day can be administered in a single dose or in several divided doses. However, it is not limited to the above-mentioned dose and administration method.

The daily dose of Compound A or a salt thereof is 20 $mg/m^2$ or more, preferably 40 $mg/m^2$ or more, more preferably 60 $mg/m^2$ or more, and still more preferably 80 $mg/m^2$ or more. The upper limit value of the daily dose is 200 $mg/m^2$, preferably 150 $mg/m^2$, more preferably 120 $mg/m^2$, and particularly preferably 100 $mg/m^2$. The daily dose is more preferably 40 to 200 $mg/m^2$, still more preferably 40 to 150 $mg/m^2$, even still more preferably 80 to 150 $mg/m^2$, and still further more preferably 80 to 120 $mg/m^2$.

In addition, as another aspect, the daily dose is preferably 20 to 120 $mg/m^2$, more preferably 40 to 120 $mg/m^2$, still more preferably 40 to 100 $mg/m^2$, and even still more preferably 60 to 100 $mg/m^2$.

By setting the dose in such a range, adverse effects can be minimized and the therapeutic effect as an antitumor agent can be maximized.

As a method of administering Compound A or a salt thereof, a course of once-weekly dosing for 3 weeks at a single dose of 20 to 200 $mg/m^2$ and then cessation of medication at the 4th week can be repeated a plurality of times. In this case, the single dose is the same as the above-mentioned daily dose, but is preferably 40 to 200 $mg/m^2$, more preferably 40 to 150 $mg/m^2$, and still more preferably 80 to 150 $mg/m^2$. In addition, as another aspect, the single dose is preferably 20 to 120 $mg/m^2$, more preferably 40 to 120 $mg/m^2$, and still more preferably 40 to 100 $mg/m^2$.

The antitumor platinum complex can be administered according to known clinical practice. Dose and dosing regimen may vary according to the particular disease symptoms and overall patient symptoms. For example, with respect to an adult patient, usually a dose of 1 to 2000 $mg/m^2$/day for an adult can be administered in a single dose or in several divided doses by oral or parenteral administration (for example, injection, drip infusion, or rectal administration). Although not particularly limited, the dose is, for example, 1 to 2000 $mg/m^2$, preferably 10 to 2000 $mg/m^2$, more preferably 50 to 1000 $mg/m^2$, and still more preferably 100 to 500 $mg/m^2$, which can be usually administered in 1 to 3 divided doses per day. In case where the patient experiences excessive toxicity, then a reduced dose will be required. Dose and dosing regimen may vary in case where one or more additional chemotherapeutic agents are used in addition to the combination therapy of the embodiment of the invention. The dosing regimen can be determined by a physician treating a particular patient.

The dose or formulation amount of the antitumor platinum complex and Compound A or the salt thereof included in the antitumor agent according to the embodiment of the invention is not particularly limited as long as it is in a range of enhancing an antitumor effect.

The amount of Compound A of the embodiment of the invention to be used varies depending on an individual combination with an antitumor platinum complex, but is, for example, about 0.0001 to 10000 times (weight ratio) and preferably about 0.001 to 1000 times (weight ratio) that of the antitumor platinum complex.

More specifically, in case where Compound A is combined with cisplatin, although not particularly limited, for example, the dose of Compound A of the embodiment of the invention is 20 to 200 $mg/m^2$/day, preferably 40 to 200 $mg/m^2$/day, more preferably 40 to 150 $mg/m^2$/day, and still more preferably 80 to 150 $mg/m^2$/day for an adult, the dose of cisplatin is 1 to 2000 $mg/m^2$/day, preferably 10 to 2000 $mg/m^2$/day, more preferably 50 to 1000 $mg/m^2$/day, and still more preferably 100 to 500 $mg/m^2$/day for an adult, and the dose of the compound of the embodiment of the invention is about 0.0001 to 10000 times (weight ratio) and preferably about 0.001 to 1000 times (weight ratio) that of cisplatin.

In addition, in another aspect, for example, the dose of cisplatin is 1 to 100 $mg/m^2$/day, preferably 5 to 50 $mg/m^2$/day, more preferably 10 to 50 $mg/m^2$/day, and still more preferably 10 to 30 $mg/m^2$/day for an adult, and the dose of the compound of the embodiment of the invention is about 0.2 to 200 times (weight ratio) and preferably about 1.6 to 15 times (weight ratio) that of cisplatin.

In addition, in case where the compound of the embodiment of the invention is combined with carboplatin, although not particularly limited, for example, the dose of Compound A of the embodiment of the invention is 20 to 200 $mg/m^2$/day, preferably 40 to 200 $mg/m^2$/day, more preferably 40 to 150 $mg/m^2$/day, and still more preferably 80 to 150 $mg/m^2$/day for an adult, the dose of carboplatin is 10 to 2000 $mg/m^2$/day, preferably 50 to 1000 $mg/m^2$/day, and more preferably 100 to 500 $mg/m^2$/day for an adult, and the dose of the compound of the embodiment of the invention is about 0.0001 to 10000 times (weight ratio), preferably about 0.001 to 1000 times (weight ratio), more preferably about 0.01 to 20 times (weight ratio), and still more preferably about 0.16 to 1.5 times (weight ratio) that of carboplatin.

In addition, in case where the compound of the embodiment of the invention is combined with oxaliplatin, although not particularly limited, for example, the dose of Compound A of the embodiment of the invention is 20 to 200 $mg/m^2$/day, preferably 40 to 200 $mg/m^2$/day, more preferably 40 to 150 $mg/m^2$/day, and still more preferably 80 to 150 $mg/m^2$/day for an adult, the dose of oxaliplatin is 10 to 2000 $mg/m^2$/day, preferably 50 to 1000 $mg/m^2$/day, and more preferably 100 to 500 $mg/m^2$/day for an adult, and the dose of the compound of the embodiment of the invention is about 0.0001 to 10000 times (weight ratio) and preferably about 0.001 to 1000 times (weight ratio) that of oxaliplatin.

In addition, in another aspect, the dose of oxaliplatin is 10 to 500 $mg/m^2$/day, preferably 50 to 500 $mg/m^2$/day, and more preferably 50 to 150 $mg/m^2$/day for an adult, and the dose of the compound of the embodiment of the invention is

about 0.04 to 20 times (weight ratio) and preferably about 0.53 to 3 times (weight ratio) that of oxaliplatin.

Examples of dosage forms of the antitumor agent according to the embodiment of the invention include a tablet, a capsule, a powder, a syrup, a granule, a pill, a suspension, an emulsion, a solution, a suppository, an eye drop, a nasal drop, an ear drop, a patch, an ointment, and an injection, among which an injection is preferred. Each of these dosage forms of administration can be produced by a formulation method conventionally known to those skilled in the art.

The antitumor agent for use in the treatment of a tumor as defined in the claims according to the embodiment of the invention is preferably an anti-malignant tumor agent and can be used as an anticancer agent.

The antitumor agent according to the embodiment of the invention can be effectively used for the treatment of various types of tumors including biliary tract cancer, breast cancer, and pancreatic cancer. Among them, biliary tract cancer is more preferable, and the antitumor agent for use according to the embodiment of the invention is particularly effective for the treatment of cholangiocarcinoma.

(Antitumor effect enhancer)

The invention also relates to an antitumor effect enhancer for use in the treatment of a tumor as defined in the claims including Compound A or a salt thereof for enhancing an antitumor effect of an antitumor platinum complex on a cancer patient as defined in the claims.

That is, according to the invention, an antitumor effect enhancer is provided for use in the treatment of a tumor as defined in the claims including 1-(2-deoxy-2-fluoro-4-thio-β-D-arabinofuranosyl)cytosine or a salt thereof, each of which is used in combination with an antitumor platinum complex.

Typically, the antitumor effect enhancer for use according to the embodiment of the invention may contain additives such as an excipient, a binder, a lubricant, a disintegrant, a coloring agent, a flavoring agent, an emulsifier, a surfactant, a solubilizing agent, a suspending agent, an isotonic agent, a buffering agent, a preservative, an antioxidant, a stabilizer, and an absorption promoter, which have been commonly used in the formulation.

The antitumor effect enhancer for use according to the embodiment of the invention can be administered to a subject simultaneously with, separately from, sequentially with, or at intervals with the antitumor platinum complex.

Parenteral administration is preferred as the route of administration of the antitumor effect enhancer according to the embodiment of the invention. For example, intravenous injections (intravenous infusions) such as drip infusion, intramuscular injection, intraperitoneal injection, subcutaneous injection, intraocular injection and intrathecal injection can be mentioned as parenteral administration. The method of administration includes administration by syringe or drip infusion.

The dose or formulation amount of Compound A or the salt thereof included in the antitumor effect enhancer according to the embodiment of the invention is not particularly limited as long as it is in a range of enhancing an antitumor effect.

The amount of Compound A of the embodiment of the invention to be used varies depending on an individual combination with the antitumor platinum complex, but is, for example, about 0.0001 to 10000 times (weight ratio) and preferably about 0.001 to 1000 times (weight ratio) that of the antitumor platinum complex.

With respect to the dose and administration method of Compound A or a salt thereof included in the antitumor effect enhancer according to the embodiment of the invention, a dose of 1 to 2000 mg/m$^2$/day can be administered in a single dose or in several divided doses. However, it is not limited to the above-mentioned dose and administration method.

The daily dose of Compound A or a salt thereof is 20 mg/m$^2$ or more, preferably 40 mg/m$^2$ or more, more preferably 60 mg/m$^2$ or more, and still more preferably 80 mg/m$^2$ or more. The upper limit value of the daily dose is 200 mg/m$^2$, preferably 150 mg/m$^2$, more preferably 120 mg/m$^2$, and particularly preferably 100 mg/m$^2$. The daily dose is more preferably 40 to 200 mg/m$^2$, still more preferably 40 to 150 mg/m$^2$, even still more preferably 80 to 150 mg/m$^2$, and still further more preferably 80 to 120 mg/m$^2$.

In addition, as another aspect, the daily dose is preferably 20 to 120 mg/m$^2$, more preferably 40 to 120 mg/m$^2$, still more preferably 40 to 100 mg/m$^2$, and even still more preferably 60 to 100 mg/m$^2$.

By setting the dose in such a range, adverse effects can be minimized and the therapeutic effect as an antitumor agent can be maximized.

As a method of administering Compound A or a salt thereof, a course of once-weekly dosing for 3 weeks at a single dose of 20 to 200 mg/m$^2$ and then cessation of medication at the 4th week can be repeated a plurality of times. In this case, the single dose is the same as the above-mentioned daily dose, but is preferably 40 to 200 mg/m$^2$, more preferably 40 to 150 mg/m$^2$, and still more preferably 80 to 150 mg/m$^2$. In addition, as another aspect, the single dose is preferably 20 to 120 mg/m$^2$, more preferably 40 to 120 mg/m$^2$, and still more preferably 40 to 100 mg/m$^2$.

The antitumor effect enhancer for use according to the embodiment of the invention is preferably an anti-malignant tumor effect enhancer and can be used as an anticancer effect enhancer.

The antitumor effect enhancer for use according to the embodiment of the invention can be effectively used for the treatment of various types of tumors including biliary tract cancer, breast cancer, and pancreatic cancer. Among them,

biliary tract cancer is more preferable, and the antitumor effect enhancer for use according to the embodiment of the invention is particularly effective for the treatment of cholangiocarcinoma.

(Antitumor kit)

According to the invention, an antitumor kit is provided for use in the treatment of a tumor as defined in the claims including a preparation including an antitumor platinum complex and a preparation including 1-(2-deoxy-2-fluoro-4-thio-β-D-arabinofuranosyl)cytosine or a salt thereof.

The antitumor kit for use according to the embodiment of the invention is a kit including a combination of an antitumor platinum complex (a) and Compound A or a salt thereof (b).

In addition, in the kit, the antitumor platinum complex (a) and Compound A or the salt thereof (b) can be in various known preparation forms, and depending on the preparation form, (a) and (b) are housed in various commonly used containers.

Further, in the kit, the antitumor platinum complex (a) and Compound A or the salt thereof (b) may be housed in separate containers or may be mixed and housed in the same container. It is preferable that the antitumor platinum complex (a) and Compound A or the salt thereof (b) are housed in separate containers.

The invention thus provides an antitumor platinum complex and 1-(2-deoxy-2-fluoro-4-thio-β-D-arabinofuranosyl)cytosine or a salt thereof for use in a method for the treatment of biliary tract cancer, breast cancer or pancreatic cancer, the method including a step of administering a therapeutically effective dose of the antitumor platinum complex and 1-(2-deoxy-2-fluoro-4-thio-β-D-arabinofuranosyl)cytosine or the salt thereof to a subject (a mammal including a human) in need of such treatment.

In addition, the invention provides a therapeutically effective dose of 1-(2-deoxy-2-fluoro-4-thio-β-D-arabinofuranosyl)cytosine or a salt thereof for use in treating a tumor as defined in the claims in case of being used in a combination therapy and a therapeutically effective dose of an antitumor platinum complex in case of being used in a combination therapy are administered in combination to a subject.

Further, the invention provides a therapeutically effective dose of 1-(2-deoxy-2-fluoro-4-thio-β-D-arabinofuranosyl)cytosine or a salt thereof for use in treating a tumor as defined in the claims in case of being used in a combination therapy and a therapeutically effective dose of an antitumor platinum complex in case of being used in a combination therapy are administered to a subject simultaneously, separately, sequentially, or at intervals.

According to the invention, it is possible to obtain 1-(2-deoxy-2-fluoro-4-thio-β-D-arabinofuranosyl)cytosine or a salt thereof for treating a tumor by administration thereof as a single dosage form with or as a dosage form separate from an antitumor platinum complex.

Examples

Hereinafter, the invention will be described in more detail with reference to Examples and Test Examples, but the invention is not limited to these Examples and the like.

(Example 1)

Methanesulfonate of 1-(2-deoxy-2-fluoro-4-thio-β-D-arabinofuranosyl)cytosine (Compound A) was synthesized by the method described in WO2013/146833A.

(Test Example 1)

Evaluation of antitumor activity against human pancreatic cancer-derived cell line SUIT-2 by combination use of cisplatin and oxaliplatin

Gemcitabine (hereinafter, also referred to as Gem), cisplatin, oxaliplatin, and methanesulfonate of Compound A were used as test substances.

Gemcitabine hydrochloride (manufactured by Plantex Co., Ltd.) dissolved in dimethyl sulfoxide (DMSO) was used as gemcitabine. Cisplatin (Cat. # 039-20091, manufactured by Wako Pure Chemical Industries, Ltd.) and oxaliplatin (Cat. # 00372, manufactured by Tokyo Chemical Industry Co., Ltd.), each of which dissolved in serum-free RPMI-1640 medium (Cat. # 11875-119, manufactured by Thermo Fisher Scientific Inc.), were used as cisplatin and oxaliplatin, respectively.

Human pancreatic cancer cell line SUIT-2 cells were subcultured in RPMI-1640 medium supplemented with 10% serum (Cat. # 10437-028, manufactured by Thermo Fisher Scientific Inc.). During this test, all cell cultures were carried out in a $CO_2$ incubator (set to 37°C and 5% $CO_2$, saturated with water vapor). The culture was diluted with a 10% serum-

supplemented medium to a cell density of 15000 cells/well/100 μL and the cells were seeded on a 96-well plate. On the next day, the culture supernatant of each well was discarded, followed by washing twice with 150 μL of serum-free medium, and 100 μL of serum-free medium was added thereto, followed by culturing for 3 days.

The methanesulfonate of Compound A and gemcitabine were dissolved in DMSO to prepare 100 mmol/L DMSO solutions thereof. The thus-prepared DMSO solutions were diluted sequentially with DMSO to prepare DMSO solutions having a final treatment concentration of 1000-fold dilution. Cisplatin and oxaliplatin were dissolved in serum-free medium to prepare a cisplatin solution 30 μmol/L and an oxaliplatin solution 60 μmol/L, respectively. The DMSO diluted solutions of Compound A and gemcitabine were diluted with the cisplatin solution (30 μmol/L) or the oxaliplatin solution (60 μmol/L) to prepare treatment liquids having a final treatment concentration of 6-fold dilution. Compound A and gemcitabine were used with a maximum concentration of 10 μmol/L at a common ratio of 1/3 and 9 concentrations in combination with cisplatin or oxaliplatin.

20 μL of Compound A or gemcitabine diluted with the cisplatin solution (30 μmol/L) or the oxaliplatin solution (60 μmol/L) was added to each well. In addition, a group in which only a solvent containing no drug was added to wells in which cells were seeded (positive control), and a group in which only a solvent containing no drug was added to wells containing only a medium (negative control) were provided. All groups were set to n = 3 well.

The cells were cultured for 3 days after the addition of the drug, and the cell viability was evaluated using a CellTiter Glo (registered trademark) Reagent (Cat. # G7570, manufactured by Promega Corporation) with the intracellular ATP level as an index. The concentration that inhibits cell viability by 50% (IC50 value) was calculated using XLFit software Ver.3 (registered trademark, manufactured by CTC Laboratory Systems Corporation).

The cell viability of each well was determined with the luminescence signal level of the negative control being 0% cell viability and the luminescence signal level of the positive control being 100% cell viability. The average value and standard deviation of the cell viability of each treatment group were calculated to prepare Table 1 and Table 2.

Results of combination test with cisplatin 5 μmol/L

[Table 1]

| Cell viability average | | | | | | | | | |
|---|---|---|---|---|---|---|---|---|---|
| Concentration (nM) | 10000.0 | 3333.3 | 1111.1 | 370.4 | 123.5 | 41.2 | 13.7 | 4.6 | 1.5 |
| Gemcitabine | 22 | 32 | 54 | 73 | 82 | 94 | 99 | 97 | 98 |
| Compound A | 30 | 27 | 26 | 28 | 28 | 33 | 47 | 80 | 96 |

Cell viability SD

| Concentration (nM) | 10000.0 | 3333.3 | 1111.1 | 370.4 | 123.5 | 41.2 | 13.7 | 4.6 | 1.5 |
|---|---|---|---|---|---|---|---|---|---|
| Gemcitabine | 1.7 | 1.5 | 2.3 | 1.3 | 1.6 | 2.9 | 6.0 | 2.6 | 2.5 |
| Compound A | 15.1 | 10.1 | 4.8 | 5.3 | 1.3 | 7.5 | 4.5 | 0.5 | 2.8 |

Results of combination test with oxaliplatin 10 μmol/L

[Table 2]

| Cell viability average | | | | | | | | | |
|---|---|---|---|---|---|---|---|---|---|
| Concentration (nM) | 10000 | 3000 | 1000 | 300 | 100 | 30 | 10 | 3 | 1 |
| Gemcitabine | 61 | 80 | 89 | 96 | 101 | 97 | 97 | 102 | 98 |
| Compound A | 38 | 39 | 38 | 41 | 46 | 60 | 87 | 93 | 94 |

Cell viability SD

| Concentration (nM) | 10000 | 3000 | 1000 | 300 | 100 | 30 | 10 | 3 | 1 |
|---|---|---|---|---|---|---|---|---|---|
| Gemcitabine | 2.8 | 3.1 | 1.0 | 2.0 | 5.1 | 1.7 | 0.9 | 3.7 | 0.8 |
| Compound A | 2.2 | 2.7 | 2.9 | 4.7 | 2.6 | 1.2 | 4.1 | 2.5 | 7.2 |

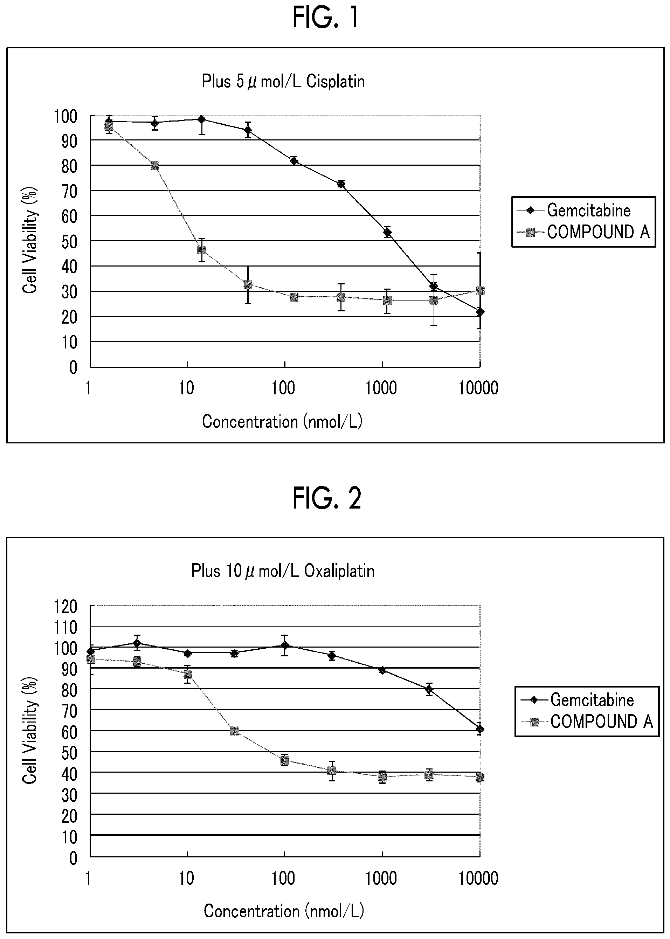

Furthermore, graphs created from Table 1 and Table 2 above are shown in Fig. 1 and Fig. 2.

The IC50 value calculated from Table 1 in a case where gemcitabine and cisplatin were used in combination was 1138.3 nmol/L (nM), and the IC50 value in a case where Compound A and cisplatin were used in combination was 12.9 nmol/L. In addition, the IC50 value calculated from Table 2 in a case where gemcitabine and oxaliplatin were used in combination was 10000 nmol/L or more, and the IC50 value in a case where Compound A and oxaliplatin were used in combination was 73.4 nmol/L. Thus, Compound A significantly enhanced the antitumor effects of cisplatin and oxaliplatin. The effect was considered to be greater than that of the existing drug, gemcitabine. A more detailed description will be given later.

On the combinational effect of the embodiment of the invention, the results of evaluation using a combination index (CI) which is a quantitative index of the combinational effect are shown. The CI can be calculated by the following equation according to Cancer Research, 2010, Vol. 70, pp. 440 to 446.

That is, in a case where the drugs to be used in combination are drug 1 and drug 2, the CI at a certain drug concentration is

CI = {(cell viability in case of using drug 1 and drug 2 in combination)/100}/{[(cell viability in case of using drug 1)/100] x [(cell viability in case of using drug 2)/100]}

CI = 1: additive effect
CI > 1: antagonistic effect
CI < 1: synergistic effect

The CI in a case where cisplatin and gemcitabine were used in combination was 0.96, and the CI in a case where cisplatin and Compound A were used in combination was 0.36. The CI in a case where oxaliplatin and gemcitabine were used in combination was 0.97, and the CI in a case where oxaliplatin and Compound A were used in combination was 0.44. Since CI < 1, the synergistic effect of the combined use of Compound A and cisplatin or oxaliplatin was observed. In addition, since it is estimated that the smaller the CI value is, the higher the synergistic effect is, it can be said that the synergistic effect of Compound A is more significant than that of the existing drug, gemcitabine.

(Test Example 2)

Evaluation of antitumor activity against human ovarian cancer cell lines ES-2 and SK-OV-3 in combination with cisplatin and carboplatin

Furthermore, the antitumor activity against human ovarian cancer-derived cell lines ES-2 and SK-OV-3 in combination with cisplatin and carboplatin was evaluated in the same manner as in Test Example 1.

Gemcitabine, cisplatin, carboplatin, and methanesulfonate of Compound A were used as test substances.

Gemcitabine hydrochloride (manufactured by Plantex Co., Ltd.) dissolved in DMSO was used as gemcitabine. Cisplatin (Cat. # 039-20091, manufactured by Wako Pure Chemical Industries, Ltd.) and carboplatin (Cat. # C2043, manufactured by Tokyo Chemical Industry Co., Ltd.), each of which dissolved in McCoy's 5a medium (Cat. # 16600-082, manufactured by Thermo Fisher Scientific Inc.) supplemented with 1% serum (Cat. # 10437-028, manufactured by Thermo Fisher Scientific Inc.), were used as cisplatin and carboplatin, respectively.

Human ovarian cancer cell line ES-2 and SK-OV-3 cells were subcultured in 10% serum-supplemented medium. During this test, all cell cultures were carried out in a $CO_2$ incubator (set to 37°C and 5% $CO_2$, saturated with water vapor). ES-2 cells were diluted with 10% serum-supplemented medium to a cell density of 10000 cells/well/100 μL and SK-OV-3 cells were diluted with 10% serum-supplemented medium to a cell density of 15000 cells/well/100 μL, and then seeded on a 96-well plate. On the next day, the culture supernatant of each well was discarded, followed by washing twice with 150 μL of 1% serum-supplemented medium, and 100 μL of 1% serum-supplemented medium was added thereto, followed by culturing for 3 days.

The methanesulfonate of Compound A and gemcitabine were dissolved in DMSO to prepare 100 mmol/L DMSO solutions thereof. The thus-prepared DMSO solutions were diluted sequentially with DMSO to prepare DMSO solutions having a final treatment concentration of 1000-fold dilution. Cisplatin and carboplatin were dissolved in 1% serum-supplemented medium to prepare a cisplatin solution 60 μmol/L and a carboplatin solution 600 μmol/L, respectively. The

DMSO diluted solutions of Compound A and gemcitabine were diluted with the cisplatin solution (60 μmol/L) or the Carboplatin solution (600 μmol/L) to prepare treatment liquids having a final treatment concentration of 6-fold dilution. Compound A and gemcitabine were used with a maximum concentration of 10 μmol/L at a common ratio of 1/3 and 9 concentrations in combination with cisplatin or Carboplatin.

20 μL of Compound A or gemcitabine diluted with the cisplatin solution (60 μmol/L) or the Carboplatin solution (600 μmol/L) was added to each well. In addition, a group in which only a solvent containing no drug was added to wells in which cells were seeded (positive control), and a group in which only a solvent containing no drug was added to wells containing only a medium (negative control) were provided. All groups were set to n = 3 well.

The cells were cultured for 3 days after the addition of the drug, and the cell viability was evaluated using a CellTiter Glo (registered trademark) Reagent (Cat. # G7570, manufactured by Promega Corporation) with the intracellular ATP level as an index. The concentration that inhibits cell viability by 50% (IC50 value) was calculated using XLFit software Ver.3 (registered trademark, manufactured by CTC Laboratory Systems Corporation).

The cell viability of each well was determined with the luminescence signal level of the negative control being 0% cell viability and the luminescence signal level of the positive control being 100% cell viability. The average value and standard deviation of the cell viability of each treatment group were calculated to prepare Tables 3 to 6.

Results of combination test with cisplatin 10 μmol/L

ES-2 cells

[Table 3]

| Cell viability average | | | | | | | | | |
|---|---|---|---|---|---|---|---|---|---|
| Concentration (nM) | 10000 | 3000 | 1000 | 300 | 100 | 30 | 10 | 3 | 1 |
| Gemcitabine | 50 | 65 | 74 | 79 | 79 | 79 | 78 | 78 | 74 |
| Compound A | 21 | 23 | 23 | 26 | 37 | 68 | 77 | 78 | 78 |

Cell viability SD

| Concentration (nM) | 10000 | 3000 | 1000 | 300 | 100 | 30 | 10 | 3 | 1 |
|---|---|---|---|---|---|---|---|---|---|
| Gemcitabine | 3.4 | 3.7 | 2.9 | 3.1 | 2.5 | 4.7 | 4.7 | 5.5 | 1.4 |
| Compound A | 0.9 | 0.7 | 0.7 | 0.5 | 1.3 | 2.3 | 0.9 | 3.2 | 2.2 |

Results of combination test with cisplatin 10 μmol/L SK-OV-3 cells

[Table 4]

| Cell viability average | | | | | | | | | |
|---|---|---|---|---|---|---|---|---|---|
| Concentration (nM) | 10000 | 3000 | 1000 | 300 | 100 | 30 | 10 | 3 | 1 |
| Gemcitabine | 30 | 59 | 79 | 83 | 83 | 83 | 84 | 84 | 82 |
| Compound A | 10 | 10 | 11 | 14 | 43 | 76 | 81 | 82 | 83 |

Cell viability SD

| Concentration (nM) | 10000 | 3000 | 1000 | 300 | 100 | 30 | 10 | 3 | 1 |
|---|---|---|---|---|---|---|---|---|---|
| Gemcitabine | 1.4 | 1.1 | 0.6 | 0.6 | 2.6 | 2.1 | 3.8 | 1.9 | 1.2 |
| Compound A | 0.5 | 0.3 | 0.4 | 0.9 | 1.6 | 3.3 | 3.0 | 1.9 | 3.2 |

Results of combination test with carboplatin 30 μmol/L

SK-OV-3 cells

[Table 5]

| Cell viability average | | | | | | | | | |
|---|---|---|---|---|---|---|---|---|---|
| Concentration (nM) | 10000 | 3000 | 1000 | 300 | 100 | 30 | 10 | 3 | 1 |
| Gemcitabine | 76 | 80 | 81 | 82 | 83 | 86 | 88 | 90 | 90 |
| Compound A | 17 | 20 | 41 | 73 | 80 | 82 | 84 | 88 | 90 |

Cell viability SD

| Concentration (nM) | 10000 | 3000 | 1000 | 300 | 100 | 30 | 10 | 3 | 1 |
|---|---|---|---|---|---|---|---|---|---|
| Gemcitabine | 5.6 | 4.9 | 1.2 | 0.9 | 0.7 | 0.5 | 1.4 | 2.9 | 1.4 |
| Compound A | 0.5 | 0.6 | 2.1 | 1.3 | 1.5 | 0.6 | 1.5 | 1.6 | 3.1 |

Results of combination test with carboplatin 100 μmol/L

ES-2 cells

[Table 6]

| Cell viability average | | | | | | | | | |
|---|---|---|---|---|---|---|---|---|---|
| Concentration (nM) | 10000 | 3000 | 1000 | 300 | 100 | 30 | 10 | 3 | 1 |
| Gemcitabine | 48 | 62 | 69 | 71 | 72 | 71 | 71 | 74 | 76 |
| Compound A | 21 | 22 | 24 | 28 | 36 | 57 | 69 | 70 | 73 |

Cell viability SD

| Concentration (nM) | 10000 | 3000 | 1000 | 300 | 100 | 30 | 10 | 3 | 1 |
|---|---|---|---|---|---|---|---|---|---|
| Gemcitabine | 1.5 | 1.8 | 1.7 | 0.7 | 1.3 | 1.6 | 1.3 | 2.5 | 2.4 |
| Compound A | 1.1 | 0.5 | 0.6 | 0.2 | 1.4 | 3.3 | 1.5 | 2.2 | 1.9 |

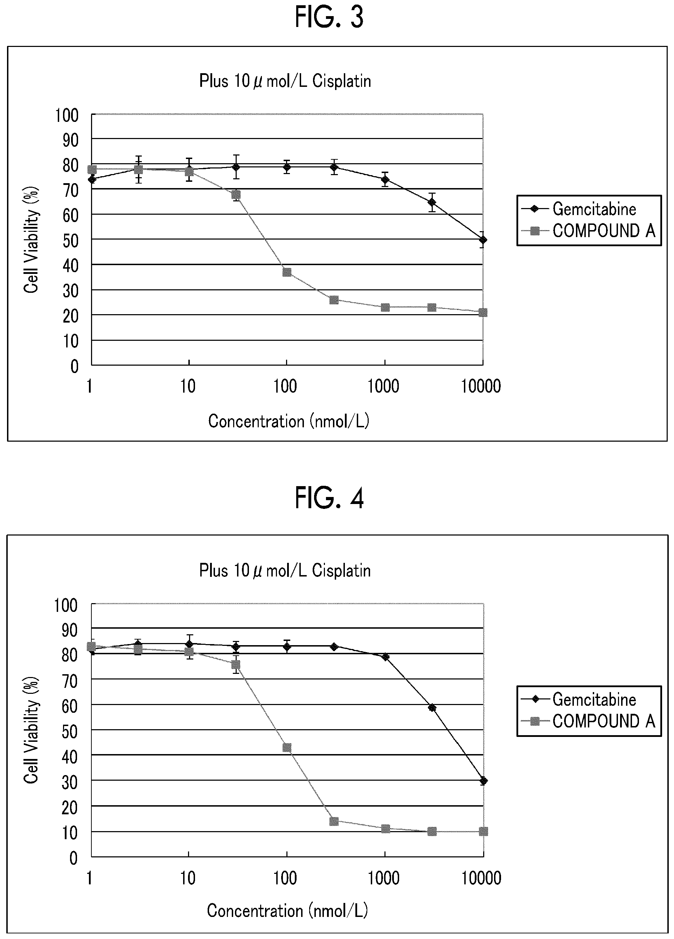

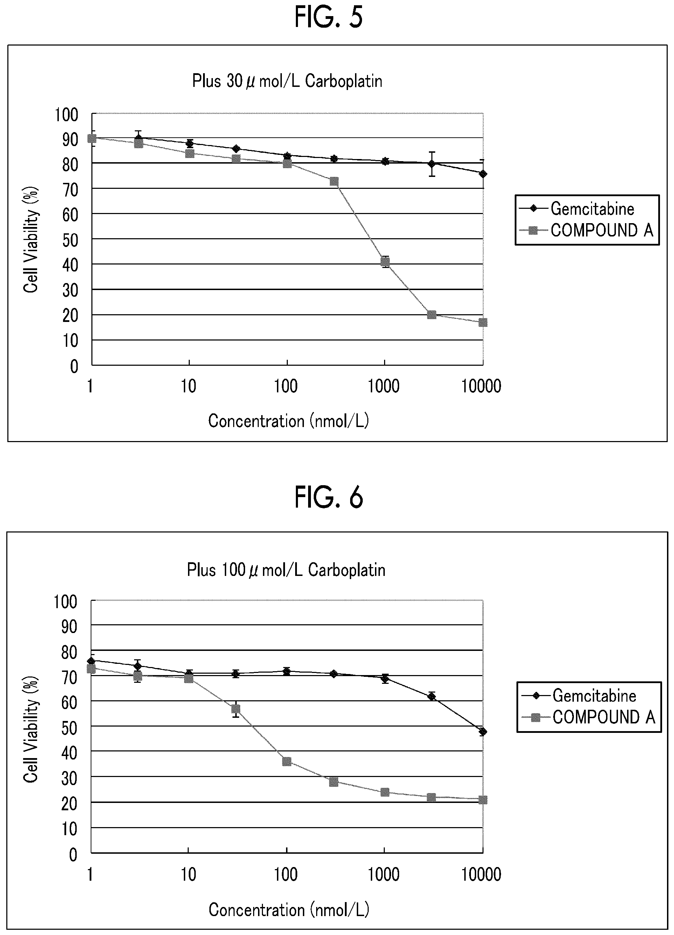

Furthermore, graphs created from Tables 3 to 6 above are shown in Figs. 3 to 6.

For ES-2 cells, the IC50 value calculated from Table 3 in a case where gemcitabine and cisplatin were used in combination could not be calculated, and the IC50 value in a case where Compound A and cisplatin were used in combination was 63.2 nmol/L. In addition, for SK-OV-3 cells, the IC50 value calculated from Table 4 in a case where gemcitabine and cisplatin were used in combination was 3797.2 nmol/L, and the IC50 value in a case where Compound A and cisplatin were used in combination was 82.2 nmol/L.

For SK-OV-3 cells, the IC50 value calculated from Table 5 in a case where gemcitabine and carboplatin were used in combination could not be calculated, and the IC50 value in a case where Compound A and carboplatin were used in combination was 676.5 nmol/L. In addition, for ES-2 cells, the IC50 value calculated from Table 6 in a case where gemcitabine and carboplatin were used in combination was 10000 nmol/L or more, and the IC50 value in a case where

Compound A and carboplatin were used in combination was 46.0 nmol/L.

The CI was calculated from Tables 3 to 6 and Table 7 was prepared as a summary.

[Table 7]

| <CI summary> | | Antitumor platinum complex | |
|---|---|---|---|
| Cell line | Drug | Cisplatin | Carboplatin |
| ES-2 Cell | Gemcitabine | 1.1 | 0.9 |
| | Compound A | 0.3 | 0.4 |
| SK-OV-3 Cell | Gemcitabine | 1.1 | 1.0 |
| | Compound A | 0.2 | 0.3 |

Since CI < 1, the synergistic effect of the combined use of Compound A and cisplatin or carboplatin was observed. In addition, since it is estimated that the smaller the CI value is, the higher the synergistic effect is, it can be said that the synergistic effect of Compound A is more significant than that of the existing drug, gemcitabine.

In each evaluation, Compound A was found to have a synergistic effect by the combined use with cisplatin or carboplatin, and the result that the synergistic effect of Compound A is more significant than that of the existing drug, gemcitabine was obtained.

(Test Example 3)

Evaluation of antitumor activity against human cholangiocarcinoma-derived cell line HuCC-T1 in combination with cisplatin

Furthermore, the antitumor activity against the human cholangiocarcinoma-derived cell line HuCC-T1 in combination with cisplatin was evaluated in the same manner as in Test Example 1.

Gemcitabine, cisplatin, and methanesulfonate of Compound A were used as test substances.

Gemcitabine hydrochloride (manufactured by Plantex Co., Ltd.) dissolved in DMSO was used as gemcitabine. Cisplatin (Cat. # 039-20091, manufactured by Wako Pure Chemical Industries, Ltd.) dissolved in serum-free RPMI-1640 medium (Cat. # 11875-119, manufactured by Thermo Fisher Scientific Inc.) was used as cisplatin.

Human cholangiocarcinoma cell line HuCC-T1 cells were subcultured in RPMI-1640 medium supplemented with 10% serum (Cat. # 10437-028, manufactured by Thermo Fisher Scientific Inc.). During this test, all cell cultures were carried out in a $CO_2$ incubator (set to 37°C and 5% $CO_2$, saturated with water vapor). The culture was diluted with a 10% serum-supplemented medium to a cell density of 15000 cells/well/100 μL and the cells were seeded on a 96-well plate. On the next day, the culture supernatant of each well was discarded, followed by washing twice with 150 μL of serum-free medium, and 100 μL of serum-free medium was added thereto, followed by culturing for 3 days.

The methanesulfonate of Compound A and gemcitabine were dissolved in DMSO to prepare 100 mmol/L DMSO solutions thereof. The thus-prepared DMSO solutions were diluted sequentially with DMSO to prepare DMSO solutions having a final treatment concentration of 1000-fold dilution. Cisplatin was dissolved in serum-free medium to prepare a cisplatin solution 60 μmol/L. The DMSO diluted solutions of Compound A and gemcitabine were diluted with the cisplatin solution (60 μmol/L) to prepare treatment liquids having a final treatment concentration of 6-fold dilution. Compound A and gemcitabine were used with a maximum concentration of 10 μmol/L at a common ratio of 1/3 and 9 concentrations in combination with cisplatin.

20 μL of Compound A or gemcitabine diluted with the cisplatin solution (60 μmol/L) was added to each well. In addition, a group in which only a solvent containing no drug was added to wells in which cells were seeded (positive control), and a group in which only a solvent containing no drug was added to wells containing only a medium (negative control) were provided. All groups were set to n = 3 well.

The cells were cultured for 3 days after the addition of the drug, and the cell viability was evaluated using a CellTiter Glo (registered trademark) Reagent (Cat. # G7570, manufactured by Promega Corporation) with the intracellular ATP level as an index. The concentration that inhibits cell viability by 50% (IC50 value) was calculated using XLFit software Ver.3 (registered trademark, manufactured by CTC Laboratory Systems Corporation).

The cell viability of each well was determined with the luminescence signal level of the negative control being 0% cell viability and the luminescence signal level of the positive control being 100% cell viability. The average value and standard deviation of the cell viability of each treatment group were calculated to prepare Table 8.

Results of combination test with cisplatin 10 μmol/L

[Table 8]

| Cell viability average | | | | | | | | | |
|---|---|---|---|---|---|---|---|---|---|
| Concentration (nM) | 10000.0 | 3333.3 | 1111.1 | 370.4 | 123.5 | 41.2 | 13.7 | 4.6 | 1.5 |
| Gemcitabine | 56 | 83 | 103 | 113 | 116 | 113 | 116 | 113 | 114 |
| Compound A | 34 | 36 | 40 | 44 | 66 | 96 | 110 | 114 | 117 |

Cell viability SD

| Concentration (nM) | 10000.0 | 3333.3 | 1111.1 | 370.4 | 123.5 | 41.2 | 13.7 | 4.6 | 1.5 |
|---|---|---|---|---|---|---|---|---|---|
| Gemcitabine | 4.3 | 3.9 | 6.0 | 1.7 | 1.1 | 4.0 | 1.6 | 1.1 | 0.3 |
| Compound A | 4.1 | 2.2 | 1.2 | 8.0 | 12.6 | 5.0 | 2.3 | 2.8 | 0.3 |

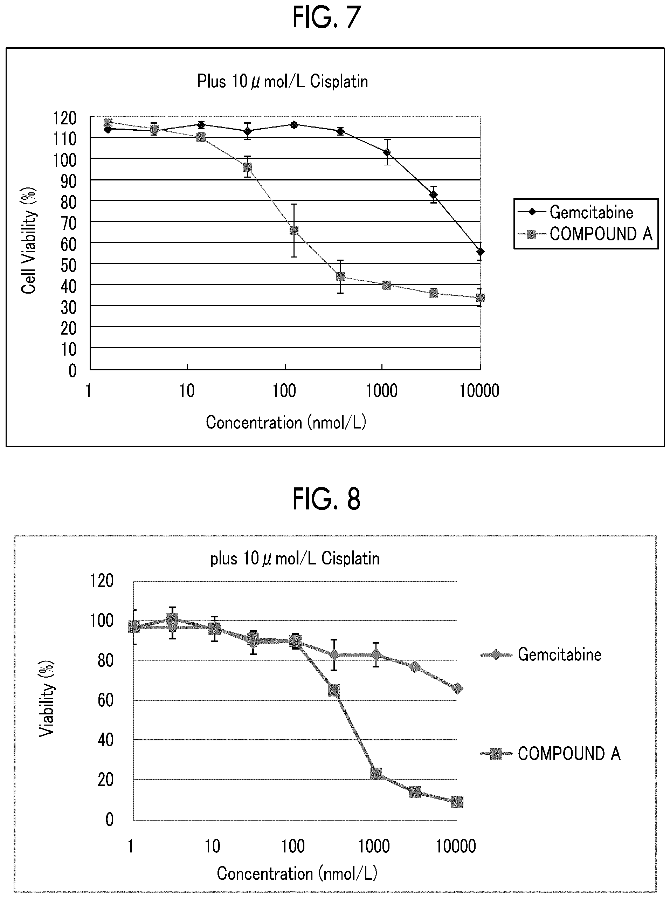

Furthermore, a graph created from Table 8 above is shown in Fig. 7.

The IC50 value calculated from Table 8 in a case where gemcitabine and cisplatin were used in combination was 10000 nmol/L or more, and the IC50 value in a case where Compound A and cisplatin were used in combination was 261.0 nmol/L. Thus, Compound A significantly enhanced the antitumor effect of cisplatin. The effect was considered to be greater than that of the existing drug, gemcitabine.

The CI in a case where cisplatin and gemcitabine were used in combination was 0.90, and the CI in a case where cisplatin and Compound A were used in combination was 0.34. Since CI < 1, the synergistic effect of the combined use of Compound A and cisplatin was observed. In addition, since it is estimated that the smaller the CI value is, the higher the synergistic effect is, it can be said that the synergistic effect of Compound A is more significant than that of the existing drug, gemcitabine.

(Test Example 4)

Evaluation of antitumor activity against cholangiocarcinoma cell line TFK-1 by combined use of cisplatin in spheroid culture

Gemcitabine, cisplatin, and methanesulfonate of Compound A were used as test substances.

Gemcitabine hydrochloride (manufactured by Plantex Co., Ltd.) dissolved in dimethyl sulfoxide (DMSO) was used as gemcitabine. Cisplatin (Cat. # 039-20091, manufactured by Wako Pure Chemical Industries, Ltd.) dissolved in DMSO was used as cisplatin.

Human cholangiocarcinoma cell line TFK-1 cells were subcultured in RPMI-1640 medium supplemented with 10% serum (Cat. # 10437-028, manufactured by Thermo Fisher Scientific Inc.). During this test, all cell cultures were carried out in a $CO_2$ incubator (set to 37°C and 5% $CO_2$, saturated with water vapor). The culture was diluted with 10% serum-supplemented medium to a cell density of 5000 cells/well/100 μL, and seeded on an Ultra-Low Attachment 96-well plate (Cat. # 7007, manufactured by Corning Incorporated). The cells were cultured for 4 days to form spheroids.

The methanesulfonate of Compound A and gemcitabine were dissolved in DMSO to prepare 10 mmol/L DMSO solutions thereof. The thus-prepared DMSO solutions were diluted sequentially with DMSO to prepare DMSO solutions having a final treatment concentration of 1000-fold dilution. Cisplatin was dissolved in DMSO to prepare a 100 mmol/L DMSO solution. The thus-prepared DMSO solution of cisplatin was diluted with DMSO to prepare a DMSO solution having a final treatment concentration of 5000-fold dilution. Further, the DMSO solution of cisplatin was diluted with 10% serum-supplemented medium to prepare a cisplatin-containing medium having a final treatment concentration of 10-fold dilution. The DMSO diluted solutions of Compound A and gemcitabine were diluted with the cisplatin-containing medium to prepare treatment liquids having a final treatment concentration of 10-fold dilution. Compound A and gemcitabine were used with a maximum concentration of 10 μmol/L at a common ratio of 1/3 and 9 concentrations in combination with cisplatin (10 μmol/L).

10 μL of Compound A or gemcitabine diluted with the cisplatin-containing medium was added to each well. In addition, a group in which only a solvent containing no drug was added to wells in which cells were seeded (positive control), and a group in which only a solvent containing no drug was added to wells containing only a medium (negative control) were provided. All groups were set to n = 3 well.

The cells were cultured for 3 days after the addition of the drug, and the cell viability was evaluated using a

CellTiter Glo (registered trademark) Reagent (Cat. # G7570, manufactured by Promega Corporation) with the intracellular ATP level as an index. The concentration that inhibits cell viability by 50% (IC50 value) was calculated using XLFit software Ver.3 (registered trademark, manufactured by CTC Laboratory Systems Corporation).

The cell viability of each well was determined with the luminescence signal level of the negative control being 0% cell viability and the luminescence signal level of the positive control being 100% cell viability. The average value and standard deviation of the cell viability of each treatment group were calculated to prepare Table 9.

Results of combination test with cisplatin 10 μmol/L

[Table 9]

| Cell viability average | | | | | | | | | |
|---|---|---|---|---|---|---|---|---|---|
| Concentration (nM) | 10000.0 | 3000.0 | 1000.0 | 300.0 | 100.0 | 30.0 | 10.0 | 3.0 | 1.0 |
| Gemcitabine | 66 | 77 | 83 | 83 | 90 | 89 | 97 | 97 | 97 |
| Compound A | 9 | 14 | 23 | 65 | 90 | 91 | 96 | 101 | 97 |

Cell viability SD

| Concentration (nM) | 10000.0 | 3000.0 | 1000.0 | 300.0 | 100.0 | 30.0 | 10.0 | 3.0 | 1.0 |
|---|---|---|---|---|---|---|---|---|---|
| Gemcitabine | 0.8 | 1.3 | 6.0 | 7.7 | 3.4 | 5.7 | 3.3 | 6.1 | 8.8 |
| Compound A | 0.3 | 0.3 | 1.5 | 2.2 | 3.9 | 3.9 | 6.2 | 6.0 | 0.5 |

Furthermore, a graph created from Table 9 above is shown in Fig. 8.

The IC50 value in a case where gemcitabine and cisplatin (10 μmol/L) were used in combination in spheroid culture was 10000 nmol/L or more, and the IC50 value in a case where Compound A and cisplatin (5 μmol/L) were used in combination was 469.3 nmol/L. Thus, Compound A significantly enhanced the antitumor effect of cisplatin. The effect was considered to be greater than that of the existing drug, gemcitabine.

The CI in a case where cisplatin and gemcitabine were used in combination was 0.78, and the CI in a case where cisplatin and Compound A were used in combination was 0.13. Since CI < 1, the synergistic effect of the combined use of Compound A and cisplatin was observed. In addition, since it is estimated that the smaller the CI value is, the higher the synergistic effect is, it can be said that the synergistic effect of Compound A is more significant than that of the existing drug, gemcitabine.

(Test Example 5)

Evaluation of antitumor activity against cholangiocarcinoma cell line HuCC-T1 by combined use of cisplatin in spheroid culture

Gemcitabine, cisplatin, and methanesulfonate of Compound A were used as test substances.

Gemcitabine hydrochloride (manufactured by Plantex Co., Ltd.) dissolved in dimethyl sulfoxide (DMSO) was used as gemcitabine. Cisplatin (Cat. # 039-20091, manufactured by Wako Pure Chemical Industries, Ltd.) dissolved in DMSO was used as cisplatin.

The cholangiocarcinoma cell line HuCC-T1 cells were subcultured in RPMI-1640 medium supplemented with 10% serum (Cat. # 10437-028, manufactured by Thermo Fisher Scientific Inc.). During this test, all cell cultures were carried out in a $CO_2$ incubator (set to 37°C and 5% $CO_2$, saturated with water vapor). The culture was diluted with 10% serum-supplemented medium to a cell density of 5000 cells/well/100 μL, and seeded on an Ultra-Low Attachment 96-well plate (Cat. # 7007, manufactured by Corning Incorporated). The cells were cultured for 4 days to form spheroids.

The methanesulfonate of Compound A and gemcitabine were dissolved in DMSO to prepare 10 mmol/L DMSO solutions thereof. The thus-prepared DMSO solutions were diluted sequentially with DMSO to prepare DMSO solutions having a final treatment concentration of 1000-fold dilution. Cisplatin was dissolved in DMSO to prepare a 100 mmol/L DMSO solution. The thus-prepared DMSO solution of cisplatin was diluted with DMSO to prepare a DMSO solution having a final treatment concentration of 5000-fold dilution. Further, the DMSO solution of cisplatin was diluted with 10% serum-supplemented medium to prepare a cisplatin-containing medium having a final treatment concentration of 10-fold dilution. The DMSO diluted solutions of Compound A and gemcitabine were diluted with the cisplatin-containing medium to prepare

treatment liquids having a final treatment concentration of 10-fold dilution. Compound A and gemcitabine were used with a maximum concentration of 10 μmol/L at a common ratio of 1/3 and 9 concentrations in combination with cisplatin (10 μmol/L).

10 μL of Compound A or gemcitabine diluted with the cisplatin-containing medium was added to each well. In addition, a group in which only a solvent containing no drug was added to wells in which cells were seeded (positive control), and a group in which only a solvent containing no drug was added to wells containing only a medium (negative control) were provided. All groups were set to n = 3 well.

The cells were cultured for 3 days after the addition of the drug, and the cell viability was evaluated using a CellTiter Glo (registered trademark) Reagent (Cat. # G7570, manufactured by Promega Corporation) with the intracellular ATP level as an index. The concentration that inhibits cell viability by 50% (IC50 value) was calculated using XLFit software Ver.3 (registered trademark, manufactured by CTC Laboratory Systems Corporation).

The cell viability of each well was determined with the luminescence signal level of the negative control being 0% cell viability and the luminescence signal level of the positive control being 100% cell viability. The average value and standard deviation of the cell viability of each treatment group were calculated to prepare Table 10.

Results of combination test with cisplatin 10 μmol/L

[Table 10]

| Cell viability average | | | | | | | | | |
|---|---|---|---|---|---|---|---|---|---|
| Concentration (nM) | 10000.0 | 3000.0 | 1000.0 | 300.0 | 100.0 | 30.0 | 10.0 | 3.0 | 1.0 |
| Gemcitabine | 50 | 73 | 79 | 84 | 88 | 90 | 94 | 96 | 94 |
| Compound A | 34 | 35 | 36 | 41 | 58 | 77 | 87 | 93 | 95 |

Cell viability SD

| Concentration (nM) | 10000.0 | 3000.0 | 1000.0 | 300.0 | 100.0 | 30.0 | 10.0 | 3.0 | 1.0 |
|---|---|---|---|---|---|---|---|---|---|
| Gemcitabine | 5.9 | 1.3 | 0.5 | 3.5 | 4.5 | 2.3 | 3.3 | 4.0 | 2.4 |
| Compound A | 2.8 | 2.1 | 2.3 | 5.8 | 1.7 | 1.4 | 6.3 | 5.8 | 1.7 |

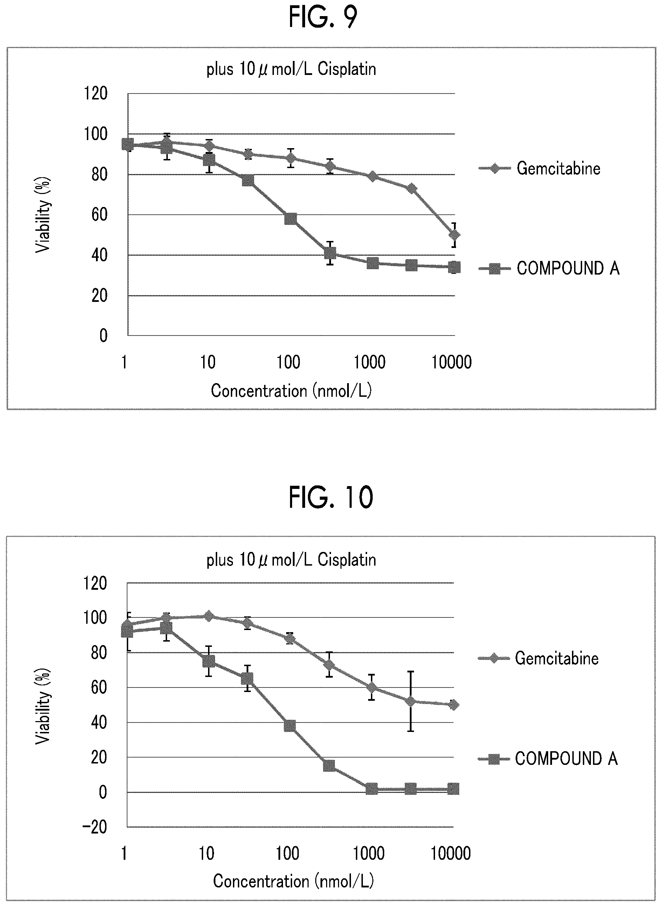

Furthermore, a graph created from Table 10 is shown in Fig. 9.

The IC50 value in a case where gemcitabine and cisplatin (10 μmol/L) were used in combination in spheroid culture was 10000 nmol/L or more, and the IC50 value in a case where Compound A and cisplatin (10 μmol/L) were used in combination was 164.6 nmol/L. Thus, Compound A significantly enhanced the antitumor effect of cisplatin. The effect was considered to be greater than that of the existing drug, gemcitabine.

The CI in a case where cisplatin and gemcitabine were used in combination was 0.79, and the CI in a case where cisplatin and Compound A were used in combination was 0.42. Since CI < 1, the synergistic effect of the combined use of Compound A and cisplatin was observed. In addition, since it is estimated that the smaller the CI value is, the higher the synergistic effect is, it can be said that the synergistic effect of Compound A is more significant than that of the existing drug, gemcitabine.

(Test Example 6)

Evaluation of antitumor activity against breast cancer-derived cell line HCC1954 by combined use of cisplatin in spheroid culture

Gemcitabine, cisplatin, and methanesulfonate of Compound A were used as test substances.

Gemcitabine hydrochloride (manufactured by Plantex Co., Ltd.) dissolved in dimethyl sulfoxide (DMSO) was used as gemcitabine. Cisplatin (Cat. # 039-20091, manufactured by Wako Pure Chemical Industries, Ltd.) dissolved in DMSO was used as cisplatin.

The human breast cancer cell line HCC1954 cells were subcultured in RPMI-1640 medium supplemented with 10% serum (Cat. # 10437-028, manufactured by Thermo Fisher Scientific Inc.). During this test, all cell cultures were carried out in a $CO_2$ incubator (set to 37°C and 5% $CO_2$, saturated with water vapor). The culture was diluted with 10% serum-supplemented medium to a cell density of 5000 cells/well/100 μL, and seeded on an Ultra-Low Attachment 96-well

plate (Cat. # 7007, manufactured by Corning Incorporated). The cells were cultured for 4 days to form spheroids.

The methanesulfonate of Compound A and gemcitabine were dissolved in DMSO to prepare 10 mmol/L DMSO solutions thereof. The thus-prepared DMSO solutions were diluted sequentially with DMSO to prepare DMSO solutions having a final treatment concentration of 1000-fold dilution. Cisplatin was dissolved in DMSO to prepare a 100 mmol/L DMSO solution. The thus-prepared DMSO solution of cisplatin was diluted with DMSO to prepare a DMSO solution having a final treatment concentration of 5000-fold dilution. Further, the DMSO solution of cisplatin was diluted with 10% serum-supplemented medium to prepare a cisplatin-containing medium having a final treatment concentration of 10-fold dilution. The DMSO diluted solutions of Compound A and gemcitabine were diluted with the cisplatin-containing medium to prepare treatment liquids having a final treatment concentration of 10-fold dilution. Compound A and gemcitabine were used with a maximum concentration of 10 μmol/L at a common ratio of 1/3 and 9 concentrations in combination with cisplatin (10 μmol/L, 5 μmol/L).

10 μL of Compound A or gemcitabine diluted with the cisplatin-containing medium was added to each well. In addition, a group in which only a solvent containing no drug was added to wells in which cells were seeded (positive control), and a group in which only a solvent containing no drug was added to wells containing only a medium (negative control) were provided. All groups were set to n = 3 well.

The cells were cultured for 3 days after the addition of the drug, and the cell viability was evaluated using a CellTiter Glo (registered trademark) Reagent (Cat. # G7570, manufactured by Promega Corporation) with the intracellular ATP level as an index. The concentration that inhibits cell viability by 50% (IC50 value) was calculated using XLFit software Ver.3 (registered trademark, manufactured by CTC Laboratory Systems Corporation).

The cell viability of each well was determined with the luminescence signal level of the negative control being 0% cell viability and the luminescence signal level of the positive control being 100% cell viability. The average value and standard deviation of the cell viability of each treatment group were calculated to prepare Table 11.

Results of combination test with cisplatin 10 μmol/L

[Table 11]

| Cell viability average | | | | | | | | | |
|---|---|---|---|---|---|---|---|---|---|
| Concentration (nM) | 10000.0 | 3000.0 | 1000.0 | 300.0 | 100.0 | 30.0 | 10.0 | 3.0 | 1.0 |
| Gemcitabine | 50 | 52 | 60 | 73 | 88 | 97 | 101 | 100 | 96 |
| Compound A | 2 | 2 | 2 | 15 | 38 | 65 | 75 | 94 | 92 |

Cell viability SD

| Concentration (nM) | 10000.0 | 3000.0 | 1000.0 | 300.0 | 100.0 | 30.0 | 10.0 | 3.0 | 1.0 |
|---|---|---|---|---|---|---|---|---|---|
| Gemcitabine | 2.2 | 17.3 | 7.3 | 6.9 | 3.1 | 3.5 | 1.7 | 2.7 | 4.4 |
| Compound A | 2.2 | 1.5 | 1.9 | 3.0 | 2.2 | 7.5 | 8.6 | 7.4 | 10.9 |

Furthermore, a graph created from Table 11 is shown in Fig. 10.

The IC50 value in a case where gemcitabine and cisplatin (10 μmol/L) were used in combination in spheroid culture was 10000 nmol/L or more, and the IC50 value in a case where Compound A and cisplatin (10 μmol/L) were used in combination was 49.4 nmol/L. Thus, Compound A significantly enhanced the antitumor effect of cisplatin. The effect was considered to be greater than that of the existing drug, gemcitabine.

The CI in a case where cisplatin and gemcitabine were used in combination was 0.60, and the CI in a case where cisplatin and Compound A were used in combination was 0.03. Since CI < 1, the synergistic effect of the combined use of Compound A and cisplatin was observed. In addition, since it is estimated that the smaller the CI value is, the higher the synergistic effect is, it can be said that the synergistic effect of Compound A is more significant than that of the existing drug, gemcitabine.

(Test Example 7)

Combinational effect test in tumor-bearing model mouse with subcutaneous transplantation of cholangiocarcinoma cell line

Gemcitabine and methanesulfonate of Compound A are used as test substances.
Gemcitabine hydrochloride (manufactured by Teva Pharmaceutical Industries Ltd.) dissolved in physiological saline was used as gemcitabine, and cisplatin (Cat. # 039-20091, manufactured by Wako Pure Chemical Industries, Ltd.) dissolved in physiological saline was used as cisplatin.
The Human cholangiocarcinoma cell line TFK-1 cells or HuCCT-1 cells are subcutaneously injected into the posterior flank of 5- to 6-week-old female BALB/cA Jcl-nu mice. After tumor transplantation, a major axis (mm) and a minor axis (mm) of the tumor are measured to calculate a tumor volume (TV). Mice are assigned to each group such that an average TV of each group is equal, and the day on which this grouping was carried out is taken as Day 1.
The dose of cisplatin is set with reference to an interview form and Cancer Genomics & Proteomics, 2012, Vol. 9, pp. 77 to 92, and the doses of gemcitabine and Compound A are set with reference to Cancer Genomics & Proteomics, 2012, Vol. 9, pp. 77 to 92 and The Journal of Pharmacology and Experimental Therapeutics, 2018, Vol. 366, pp. 125 to 135. The test liquid for the cisplatin alone group is prepared such that the administration dose is 1 to 10 mg/kg/day. In addition, the test liquid for the Compound A alone group is prepared such that the administration dose is 30 to 240 mg/kg/day. Compound A is administered through the tail vein of the mouse once a week for a total of 3 times from Day 1, and cisplatin is administered by intraperitoneal administration to the mouse once a week for a total of 3 times from Day 1. In the combined administration group, Compound A is administered at a dose of 30 to 240 mg/kg/day and cisplatin is administered at a dose of 1 to 10 mg/kg/day.
As a comparative experiment, gemcitabine is used as a control drug. The test liquid for the gemcitabine alone group is prepared such that the administration dose is 30 to 240 mg/kg/day. Gemcitabine is administered through the tail vein of the mouse once a week for a total of 3 times from Day 1, and in the combined administration group, gemcitabine is administered at a dose of 30 to 240 mg/kg/day and cisplatin is administered at a dose of 1 to 10 mg/kg/day.
In this test, doses of Compound A and gemcitabine are set using a maximum tolerable dose (MTD) of each drug. Cisplatin is used at the maximum dose that can be tolerated in combination with each drug. An antitumor agent exhibits that the dose exhibiting the maximum drug efficacy is very close to a dose expressing toxicity, and the antitumor agent is generally evaluated in the vicinity of MTD in order to evaluate a maximum antitumor effect possessed by the drug in an animal model. In this test example, the MTD and the maximum effect dose are almost synonymous.
TV in each drug administration group was measured as an index of antitumor effect, and a relative tumor volume (RTV) relative to that of Day 1 and T/C (%) were calculated by the following expression to evaluate the antitumor effect. The evaluation of the combinational effect is judged as having a combinational effect in a case where the average RTV value of the combined administration group is statistically significantly smaller (Welch's IUT, over all maximum $p < 0.05$) than the average RTV value of the individual single administration groups.

$$\text{TV (mm}^3\text{)} = (\text{major axis x minor axis}^2)/2$$

$$\text{RTV} = (\text{TV on day of tumor measurement})/(\text{TV on Day 1})$$

$$\text{T/C (\%)} = [(\text{average RTV value of test liquid administration group})/(\text{average RTV value of control group})] \times 100$$

(Test Example 8)

Combinational effect test of Compound A and antitumor platinum complex in cancer patients

<Preparation of liquid pharmaceutical composition>

The methanesulfonate of Compound A is dissolved in an appropriate amount of water for injection, and the pH is adjusted using a 1 mol/L aqueous sodium hydroxide solution. An appropriate amount of water for injection is added and mixed therewith such that the concentration of Compound Ais 20 mg/mL.
In addition, glycerin (manufactured by Merck & Co., Inc., molecular weight: 92) is added to a concentration of 1.5% by mass. The pH of this liquid pharmaceutical preparation is 2.9, and this liquid can be filtered through a membrane filter (0.22 μm) to obtain a liquid pharmaceutical preparation.

<Administration and determination of therapeutic effect>

A dosing cycle is repeated for cancer patients, in which Compound A and cisplatin are administered by intravenous injection once a week from the 1st week to the 2nd week and no medication is given at the 3rd week. Specifically, Compound A and cisplatin are administered on the 1st day and the 8th day with 21 days as one cycle, and this 21-day cycle is repeated. The dose of Compound A per administration is 8 to 135 mg/m$^2$, and the dose of cisplatin is 10 to 50 mg/m$^2$.

The therapeutic effect can be determined according to the following standards.

The evaluation target was confirmed by diagnostic imaging by magnetic resonance imaging (MRI) and determined according to the following standards.

Complete Response (CR): a state in which a tumor has completely disappeared
Partial Response (PR): a state in which the sum of tumor sizes has decreased by 30% or more
Stable Disease (SD): a state in which the size of the tumor does not change
Progressive Disease (PD): a state in which the sum of tumor sizes has increased by 20% or more and the absolute value of the tumor size has increased by 5 mm or more, or a state in which a new lesion has appeared

The invention is useful as an antitumor agent, an antitumor kit, and an antitumor effect enhancer for use in treating a tumor as defined in the claims which exhibit a significant antitumor effect.

## Claims

**1.** An antitumor agent for use in the treatment of a tumor, comprising:

an antitumor platinum complex; and
1-(2-deoxy-2-fluoro-4-thio-β-D-arabinofuranosyl)cytosine or a salt thereof,
wherein the tumor is biliary tract cancer, breast cancer, or pancreatic cancer.

**2.** The antitumor agent for use according to claim 1,
wherein the antitumor platinum complex is at least one selected from the group consisting of cisplatin, oxaliplatin, and carboplatin.

**3.** The antitumor agent for use according to claim 1 or 2,
wherein the biliary tract cancer is cholangiocarcinoma.

**4.** An antitumor effect enhancer for use in treating a tumor, comprising:

1-(2-deoxy-2-fluoro-4-thio-β-D-arabinofuranosyl)cytosine or a salt thereof, each of which is used in combination with an antitumor platinum complex,
wherein the tumor is biliary tract cancer, breast cancer, or pancreatic cancer.

**5.** An antitumor kit for use in treating a tumor comprising:

a preparation including an antitumor platinum complex; and
a preparation including 1-(2-deoxy-2-fluoro-4-thio-β-D-arabinofuranosyl)cytosine or a salt thereof,
wherein the tumor is biliary tract cancer, breast cancer, or pancreatic cancer.

**6.** An antitumor agent for use in treating a tumor, comprising:

1-(2-deoxy-2-fluoro-4-thio-β-D-arabinofuranosyl)cytosine or a salt thereof, each of which is used in combination with an antitumor platinum complex,
wherein the tumor is biliary tract cancer, breast cancer, or pancreatic cancer.

## Patentansprüche

1. Antitumormittel zur Verwendung bei der Behandlung eines Tumors, umfassend:

   ein Antitumorplatinkomplex; und
   1-(2-Desoxy-2-fluor-4-thio-β-D-arabinofuranosyl)cytosin oder ein Salz davon, wobei
   der Tumor Gallengangskrebs, Brustkrebs oder Pankreaskrebs ist.

2. Antitumormittel zur Verwendung nach Anspruch 1,
   wobei der Antitumorplatinkomplex mindestens eines ist, das aus der Gruppe, die aus Cisplatin, Oxaliplatin und Carboplatin besteht, ausgewählt ist.

3. Antitumormittel zur Verwendung nach Anspruch 1 oder 2,
   wobei der Gallengangskrebs Cholangiokarzinom ist.

4. Antitumorwirkungsverstärker zur Verwendung bei Behandlung eines Tumors, umfassend:

   1-(2-Desoxy-2-fluor-4-thio-β-D-arabinofuranosyl)cytosin oder ein Salz davon, wobei
   jedes davon in Kombination mit einem Antitumorplatinkomplex verwendet wird,
   wobei der Tumor Gallengangskrebs, Brustkrebs oder Pankreaskrebs ist.

5. Antitumor-Kit zur Verwendung bei Behandlung eines Tumors, umfassend:

   eine Zubereitung, die einen Antitumorplatinkomplex enthält; und
   eine Zubereitung, die 1-(2-Desoxy-2-fluor-4-thio-β-D-arabinofuranosyl)cytosin oder ein Salz davon enthält,
   wobei der Tumor Gallengangskrebs, Brustkrebs oder Pankreaskrebs ist.

6. Antitumormittel zur Verwendung bei Behandlung eines Tumors, umfassend:

   1-(2-Desoxy-2-fluor-4-thio-β-D-arabinofuranosyl)cytosin oder ein Salz davon, wobei
   jedes davon in Kombination mit einem Antitumorplatinkomplex verwendet wird,
   wobei der Tumor Gallengangskrebs, Brustkrebs oder Pankreaskrebs ist.

## Revendications

1. Agent antitumoral à usage dans le traitement d'une tumeur, comprenant :

   un complexe de platine antitumoral ; et
   1-(2-désoxy-2-fluoro-4-thio-β-D-arabinofuranosyle)cytosine ou un sel de celui-ci, dans lequel la tumeur est un cancer des voies biliaires, un cancer du sein ou un cancer du pancréas.

2. Agent antitumoral à usage selon la revendication 1,
   dans lequel le complexe de platine antitumoral est au moins l'un choisi parmi le groupe constitué de cisplatine, d'oxaliplatine et de carboplatine.

3. Agent antitumoral à usage selon la revendication 1 ou la revendication 2,
   dans lequel le cancer des voies biliaires est un cholangiocarcinome.

4. Amplificateur d'effet antitumoral à usage dans le traitement d'une tumeur, comprenant :

   1-(2-désoxy-2-fluoro-4-thio-β-D-arabinofuranosyle)cytosine ou un sel de celui-ci,
   chacun étant utilisé en combinaison avec un complexe de platine antitumoral, dans lequel la tumeur est un cancer des voies biliaires, un cancer du sein ou un cancer du pancréas.

5. Kit antitumoral à usage dans le traitement d'une tumeur comprenant :

une préparation incluant un complexe de platine antitumoral ; et
une préparation incluant 1-(2-désoxy-2-fluoro-4-thio-β-D-arabinofuranosyle)cytosine ou un sel de celui-ci, dans lequel la tumeur est un cancer des voies biliaires, un cancer du sein ou un cancer du pancréas.

**6.** Agent antitumoral à usage dans le traitement d'une tumeur, comprenant :
1-(2-désoxy-2-fluoro-4-thio-β-D-arabinofuranosyle)cytosine ou un sel de celui-ci, chacun étant utilisé en combinaison avec un complexe de platine antitumoral, dans lequel la tumeur est un cancer des voies biliaires, un cancer du sein ou un cancer du pancréas.

# FIG. 1

Plus 5 μ mol/L Cisplatin
Cell Viability (%)
100
90
80
70
60
50
40
30
20
10
0
1
10
100
1000
10000
Concentration (nmol/L)
Gemcitabine
COMPOUND A

# FIG. 2

Plus 10 μ mol/L Oxaliplatin
Cell Viability (%)
120
110
100
90
80
70
60
50
40
30
20
10
0
1
10
100
1000
10000
Concentration (nmol/L)
Gemcitabine
COMPOUND A

# FIG. 3

Plus 10 μ mol/L Cisplatin
Cell Viability (%)
100
90
80
70
60
50
40
30
20
10
0
1
10
100
1000
10000
Concentration (nmol/L)
Gemcitabine
COMPOUND A

# FIG. 4

Plus 10 μ mol/L Cisplatin
Cell Viability (%)
100
90
80
70
60
50
40
30
20
10
0
1
10
100
1000
10000
Concentration (nmol/L)
Gemcitabine
COMPOUND A

# FIG. 5

Plus 30 μ mol/L Carboplatin
Cell Viability (%)
100
90
80
70
60
50
40
30
20
10
0
1
10
100
1000
10000
Concentration (nmol/L)
Gemcitabine
COMPOUND A

# FIG. 6

Plus 100 μ mol/L Carboplatin
Cell Viability (%)
100
90
80
70
60
50
40
30
20
10
0
1
10
100
1000
10000
Concentration (nmol/L)
Gemcitabine
COMPOUND A

# FIG. 7

Plus 10 μ mol/L Cisplatin
Cell Viability (%)
120
110
100
90
80
70
60
50
40
30
20
10
0
1
10
100
1000
10000
Concentration (nmol/L)
Gemcitabine
COMPOUND A

# FIG. 8

plus 10 μ mol/L Cisplatin
Viability (%)
120
100
80
60
40
20
0
1
10
100
1000
10000
Concentration (nmol/L)
Gemcitabine
COMPOUND A

FIG. 9

plus 10 μ mol/L Cisplatin
Viability (%)
120
100
80
60
40
20
0
1
10
100
1000
10000
Concentration (nmol/L)
Gemcitabine
COMPOUND A

FIG. 10

plus 10 μ mol/L Cisplatin
Viability (%)
120
100
80
60
40
20
0
-20
1
10
100
1000
10000
Concentration (nmol/L)
Gemcitabine
COMPOUND A

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 1997038001 A
- WO 2013146833 A
- WO 2011074484 A
- WO 2014027658 A
- WO 2013100014 A


### Non-patent literature cited in the description


- *Cancer Letters*, 1999, vol. 144, 177-182
- *Oncology Reports*, 2002, vol. 9, 1319-1322
- *YAKUGAKU ZASSHI*, 2008, vol. 128, 307-316
- *Journal of Clinical Oncology*, 2006, vol. 24, 3946-3952
- *Journal of Clinical Oncology*, 2009, vol. 23, 3778-3785
- *Cancer Research, Proc. Amer. Assoc. Cancer Res.*, 2004, vol. 45
- *Journal of Organic Chemistry*, 1999, vol. 64, 7912-7920
- *Cancer Research*, 2010, vol. 70, 440-446
- *Cancer Genomics & Proteomics*, 2012, vol. 9, 77-92
- *The Journal of Pharmacology and Experimental Therapeutics*, 2018, vol. 366, 125-135